(19)

Europäisches
Patentamt
European
Patent Office
Office européen
des brevets

(11) **EP 4 141 880 A1**

(12) **EUROPEAN PATENT APPLICATION**

(43) Date of publication:
01.03.2023 Bulletin 2023/09

(21) Application number: **22192281.8**

(22) Date of filing: **26.08.2022**

(51) International Patent Classification (IPC):
**G16H 20/17** (2018.01)        **G16H 40/63** (2018.01)
**A61M 5/14** (2006.01)

(52) Cooperative Patent Classification (CPC):
**G16H 20/17; A61M 5/14; G16H 40/63**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **30.08.2021 US 202163238291 P**

(71) Applicant: **Insulet Corporation
Acton MA 01720 (US)**

(72) Inventors:
• **LEE, Joon Bok
Acton (US)**
• **D'ARCO, John
Wilmington (US)**
• **NAZZARO, David
Groveland (US)**
• **ZHENG, Yibin
Hartland (US)**
• **O'CONNOR, Jason
Acton (US)**

(74) Representative: **Peterreins Schley
Patent- und Rechtsanwälte PartG mbB
Hermann-Sack-Straße 3
80331 München (DE)**

(54) **COMPENSATING FOR CHANGES IN POTENCY OF A MEDICAMENT IN A MEDICAMENT DELIVERY DEVICE**

(57)    The exemplary embodiments may account for a change in potency of a medicament and adjust the dosage of medicament delivered to a user via a medicament delivery device to compensate for the change in potency. The medicament delivery device may determine the amount of change in the potency in the medicament and may make the adjustment in dosage of the medicament delivered to the user automatically without user input. The net result in that the dosage of medicament delivered to the user is better matched to the user's true need for the medicament.

**EP 4 141 880 A1**

**Description**

## BACKGROUND

The potency of a medicament may change over time. Time and/or other factors, like temperature, may change the potency of the medicament. For example, the potency of insulin may change with temperature over time. Insulin ideally is stored in a temperature range between 25° C to 30° C (77° F to 86° F). When insulin is stored at a temperature above this temperature, the potency decreases as time elapses.

This can be problematic for insulin delivery devices, like insulin pumps. These insulin delivery devices calculate basal insulin dosages based on the assumption that the potency of the insulin is constant over time and for all temperatures. However, this assumption may not hold true in many instances. For on-body insulin delivery devices, the temperature of the insulin stored therein may approach normal body temperature of 98.6° F. Given this heightened temperature relative to the ideal temperature range for storage, the potency of the insulin may decrease over time, and the insulin delivery device may under-deliver the amount of insulin needed to satisfy a user's needs due to the resulting decrease in potency.

## SUMMARY

In accordance with a first inventive aspect, a method is performed by a processor of an electronic device. The method includes calculating a basal dosage of a medicament to be delivered to a user by a medicament delivery device. The method further includes determining an amount that at least one factor affects a potency of the medicament and adjusting the calculated basal dosage of medicament to compensate for the determined amount that the at least one factor has affected the potency of the medicament.

The medicament may be insulin. The at least one factor may include temperature and/or a duration of time that the medicament has been stored in the medicament delivery device. Other factors that affect potency (e.g., exposure to light, exposure to air, etc.) may also be taken into account. The at least one factor may decrease the potency of the medicament, and the calculated dosage may be adjusted to a larger dosage to compensate. The adjusting may include adjusting the calculated basal dosage based on how much a current temperature is above a target temperature. The adjusting may comprise adjusting the calculated basal dosage based on how much an actual blood glucose level compares with a projected blood glucose level.

In accordance with another inventive aspect, a medicament delivery device includes a medicament storage and a pump for pumping medicament out of the storage for delivery to a user. The medicament delivery device also includes a storage medium for storing data and computer programming instructions. The medicament delivery device includes a processor for executing the computer programming instructions. The computer programming instructions cause the processor to calculate a basal dosage of a medicament to be delivered to a user by the medicament delivery device. The computer programming instructions also cause the processor to determine an amount that at least one factor affects a potency of the medicament and to adjust the calculated basal dosage of medicament to compensate for the determined amount that the at least one factor has affected the potency of the medicament.

The computer programming instructions may additionally cause the processor to control the pump to deliver the adjusted basal dosage of medicament to the user. The medicament may be insulin. The at least one factor may comprise temperature, exposure to light or exposure to air. The at least one factor may comprise a duration of time that the medicament has been stored in the medicament delivery device. The at least one factor may include temperature, an average temperature or a histogram of temperatures and a duration of time that the medicament has been stored in the medicament delivery device or a duration of time that the medicament has been stored in the medicament delivery device at a particular temperature or at an average temperature. The adjusting may include adjusting the calculated basal dosage based on how much a current temperature or an average temperature is above a target temperature. The adjusting may include adjusting the calculated basal dosage based on how much an actual blood glucose level compares with a projected blood glucose level.

In accordance with a further inventive aspect, a method is performed by a processor in an on-body insulin delivery device. The method includes calculating a basal dosage for a user based on at least a current blood glucose level. The method also includes calculating how much a potency of the insulin has decreased over a period and increasing the calculated basal dosage of the insulin to offset the calculated decrease in potency of the insulin.

The calculating of how much the potency of the insulin has decreased over the period may include determining a temperature for the insulin and adjusting for the determined temperature if the determined temperature exceeds a threshold. The method may further include calculating a delta of how much the determined temperature exceeds the threshold, and a magnitude of the adjustment for the determined temperature may be based on a magnitude of the delta. The calculating of how much the potency of the insulin has decreased over the period may entail determining a delta between a projected blood glucose level for the user and an actual blood glucose level for the user and determining how much the potency of the insulin has decreased based on a magnitude of the determined delta.

## BRIEF DESCRIPTION OF THE DRAWINGS

Figure 1 depicts an illustrative medicament delivery system that is suitable for practicing exemplary embodiments.

Figure 2 depicts an example of an illustrative medicament device in place on a user that is suitable for the exemplary embodiments.

Figure 3 depicts an illustrative medicament device suitable for the exemplary embodiments.

Figure 4 depicts a flowchart of illustrative steps that may be performed to realize basal medicament delivery in exemplary embodiments.

Figure 5A depicts a flowchart of illustrative steps that may be performed to adjust a basal medicament dosage due to change in potency due to duration in exemplary embodiments.

Figure 5B depicts a flowchart of illustrative steps that may be performed in an example of adjusting the basal medicament dosage based on duration.

Figure 6 depicts a diagram of different types of medicaments that may be used in exemplary embodiments.

Figure 7A depicts a flowchart of illustrative steps that may be performed to adjust a basal medicament dosage due to change in potency due to duration and temperature in exemplary embodiments.

Figure 7B depicts a flowchart of illustrative steps that may be performed in an example of adjusting the basal medicament dosage based on duration and temperature.

Figure 8A depicts a flowchart of illustrative steps that may be performed to adjust a basal medicament dosage due to change in potency based on a delta between projected analyte level versus an actual analyte level in exemplary embodiments.

Figure 8B depicts a flowchart of illustrative steps that may be performed in an example of adjusting the basal medicament dosage based on the delta between projected analyte level versus an actual analyte level in exemplary embodiments.

## DETAILED DESCRIPTION

The exemplary embodiments may account for a change in potency of a medicament and adjust the dosage of medicament delivered to a user via a medicament delivery device to compensate for the change in potency. The medicament delivery device may determine the amount of change in the potency in the medicament and may make adjustments in dosage of the medicament delivered to the user automatically without user input. The net result is that the dosage of medicament delivered to the user is better matched to the user's true needs for the medicament.

The exemplary embodiments may account for different factors that affect potency. For example, the exemplary embodiments may account for duration of time that a medicament has been in an environment that affects potency. The exemplary embodiments may also account for temperature as a factor that affects potency of the medicament. For instance, when a temperature is above a threshold, the potency of the medicament may be affected. The exemplary embodiments may account for multiple such factors jointly affecting the potency of the medicament, including other factors, such as exposure to air and/or exposure to light. The dosage of medicament delivered to the user may be increased when the potency decreases and decreased when the potency increases.

In some embodiments, the medicament is insulin, and the medicament delivery device is an insulin delivery device, such as an insulin pump. In these embodiments, the control system of the insulin delivery device may determine changes in potency of the insulin and compensate for the changes in potency when calculating dosages, such as basal dosages, that are delivered to the user. The control system may determine changes in potency of the insulin based on how long the insulin has been stored in the drug delivery device or how long the insulin has been at a heightened temperature that is above a threshold temperature. Moreover, the magnitude of change in the potency may be proportional to how much the temperature exceeds the threshold temperature. Alternatively, the changes in potency may be determined based on actual analyte levels versus projected analyte levels. For example, the difference between actual glucose levels and projected glucose levels may be analyzed to identify changes in potency.

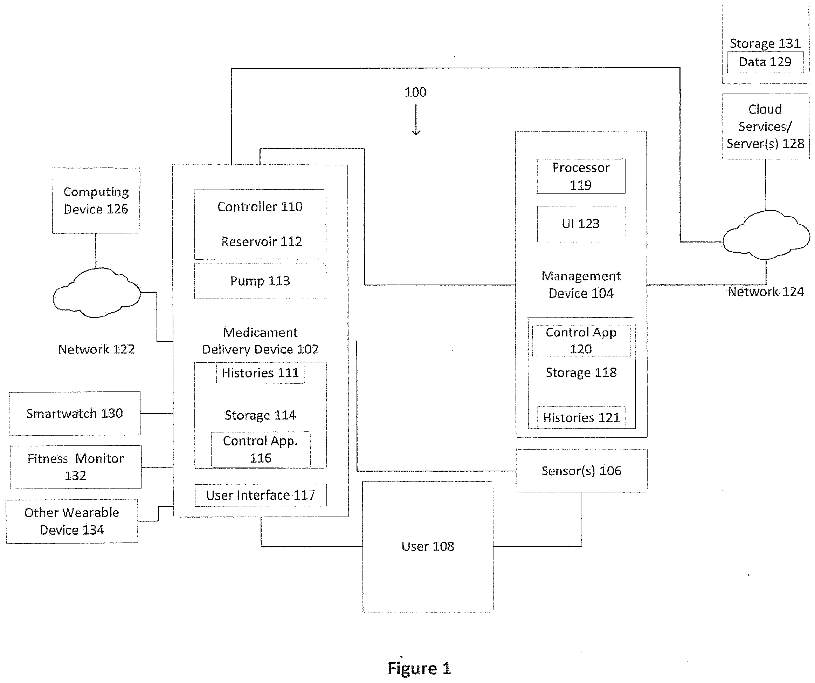

Figure 1 depicts an illustrative medicament delivery system 100 that is suitable for delivering a medicament, such as insulin, a GLP-1 agonist, pramlintide or other medicament like those detailed below, to a user 108 in accordance with exemplary embodiments. The medicament delivery system 100 includes a medicament delivery device 102. The medicament delivery device 102 may be a wearable device that is worn on the body of the user 108. The medicament delivery device 102 may be directly coupled to a user (e.g., directly attached to a body part and/or skin of the user 108 via an adhesive or the like). In an example, a surface of the medicament delivery device 102 may include an adhesive to facilitate attachment to the user 108.

The medicament delivery device 102 may include a controller 110. The controller 110 may be implemented in hardware, software, or any combination thereof. The controller 110 may, for example, be a microprocessor, a logic circuit, a field programmable gate array (FPGA), an application specific integrated circuit (ASIC), etc. The controller 110 may maintain a date and time as well as other functions (e.g., calculations or the like). The controller 110 may be operable to execute a control application 116 stored in the storage 114 that enables the controller 110 to implement a control system for controlling operation of the medicament delivery device 102. The control application 116 may control medicament delivery to the user 108 as described herein. The storage 114 may hold histories 111 for a user, such as a history of automated basal medicament deliveries, a history of bolus medicament deliveries, meal event history, activity event history, sensor data and the like. In addition, the controller 110 may be operable to receive data or information. The storage 114 may include both primary memory and secondary memory. The storage 114 may include random access memory (RAM), read only memory (ROM), optical storage, magnetic storage, removable storage media, solid state storage or the like.

The medicament delivery device 102 may include a reservoir 112 for storing medicament for delivery to the user 108 as warranted. A fluid path to the user 108 may be provided, and the medicament delivery device 102 may expel the medicament from the reservoir 112 to deliver the medicament to the user 108 via the fluid path. The fluid path may, for example, include tubing coupling the medicament delivery device 102 to the user 108 (e.g., tubing coupling a cannula to the reservoir 112).

There may be one or more communications links with one or more devices physically separated from the medicament delivery device 102 including, for example, a management device 104 of the user and/or a caregiver of the user and/or sensor(s) 106. In some embodiments, no management device 104 is provided. The communication links may include any wired or wireless communication link operating according to any known communications protocol or standard, such as Bluetooth®, Wi-Fi, a near-field communication standard, a cellular standard or another wireless protocol. The medicament delivery device 102 also may include a user interface 117, such as an integrated display device for displaying information to the user 108 and in some embodiments, receiving information from the user 108. The user interface 117 may include a touchscreen and/or one or more input devices, such as a button, a knob or a keyboard.

The medicament delivery device 102 may interface with a network 122. The network 122 may include a local area network (LAN), a wide area network (WAN) or a combination therein. A computing device 126 may be interfaced with the network, and the computing device may communicate with the medicament delivery device 102.

The medicament delivery system 100 may include sensor(s) 106 for sensing the levels of one or more analytes. The sensor(s) 106 may be coupled to the user 108 by, for example, adhesive or the like and may provide information or data on one or more medical conditions and/or physical attributes of the user 108. The sensor(s) 106 may, in some exemplary embodiments provide periodic glucose concentration measurements and may be a continuous glucose monitor (CGM), or another type of device or sensor that provides glucose level measurements. The sensor(s) 106 may be physically separate from the medicament delivery device 102 or may be an integrated component thereof. The sensor(s) 106 may provide the controller 110 with data indicative of one or more measured or detected analyte levels of the user 108. The information or data provided by the sensor(s) 106 may be used to adjust medicament delivery operations of the medicament delivery device 102.

The medicament delivery system 100 may also include the management device 104. In some embodiments, no management device 104 is needed; rather the medicament delivery device 102 may manage itself. The management device 104 may be a special purpose device, such as a dedicated personal diabetes manager (PDM) device. The management device 104 may be a programmed general-purpose device, such as any portable electronic device including, for example, a dedicated controller, such as a processor, a microcontroller, or the like. The management device 104 may be used to program or adjust operation of the medicament delivery device 102 and/or the sensor(s)106. The management device 104 may be any portable electronic device including, for example, a customized handheld electronic device, a smartphone, a smartwatch, or a tablet. In the depicted example, the management device 104 may include a processor 119 and a storage 118. The processor 119 may execute processes to manage and control the delivery of the medicament to the user 108. The processor 119 may also be operable to execute programming code stored in the storage 118. For example, the storage 118 may be operable to store one or more control applications 120 for execution by the processor 119. The one or more control applications 120 may be responsible for controlling the medicament delivery device 102, e.g., the automatic insulin delivery (AID) delivery of insulin to the user 108. The storage 118 may store the one or more control applications 120, histories 121 like those described above for the medicament delivery device 102 and other data and/or programs.

The management device 104 may include a user interface (UI) 123 for communicating with the user 108. The user interface 123 may include a display, such as a touchscreen, for displaying information. The touchscreen may also be used to receive input when it is a touch screen. The user interface 123 may also include input elements, such as a keyboard, button, knob or the like.

The management device 104 may interface with a network 124, such as a LAN or WAN or combination of such networks. The management device 104 may communicate over network 124 with one or more servers or cloud services 128.

Other devices, like smartwatch 130, fitness monitor 132 and other wearable device 134 may be part of the medicament delivery system 100. These devices 130, 132 and 134 may communicate with the medicament delivery device 102 to receive information and/or issue commands to the medicament delivery device 102. These devices 130, 132 and 134 may execute computer programming instructions to perform some of the control functions otherwise performed by controller 110 or processor 119. These devices 130, 132 and 134 may include displays for displaying information, e.g., analyte levels like current blood glucose level, medicament on board, medicament delivery history, etc. The displays may show a user interface for providing input, such as to request a change in basal medicament dosage or delivery of a bolus of medicament. These devices 130, 132 and 134 may also have wireless communication connections with the sensor 106 to directly receive analyte data.

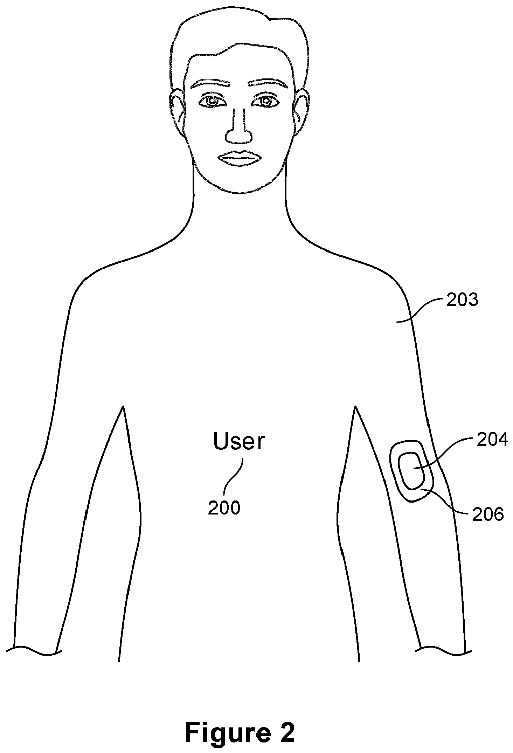

Figure 2 shows an example of a medicament delivery device 204 suitable for exemplary embodiments that is secured to a user 200. In this illustrative example, the medicament delivery device 204 is a patch pump. The medicament delivery device 204 is secured to the back of the arm 203 of the user 200 via an adhesive pad 206. The back of the arm 203 is one location that is well suited for such a device and is a non-intrusive and discreet location for the user 200.

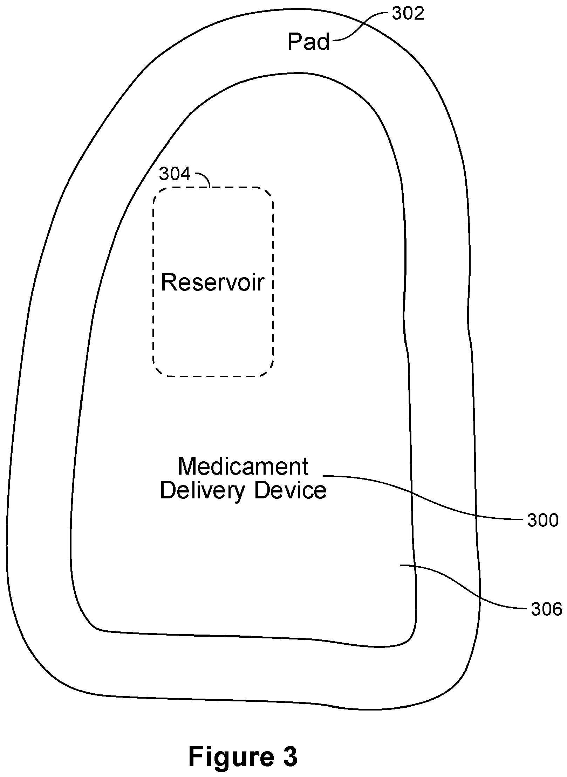

Figure 3 shows some additional detail of the medicament delivery device 300. The medicament delivery device 300 may include one or more housing components 306. Inside the one or more housing components 306 may be a reservoir 304 for storing the medicament. The adhesive pad 302 is secured to the underside of the medicament delivery device 300. The adhesive pad 302 contains an adhesive for securing the medicament delivery device 300 to the skin of the user 108.

Because the medicament delivery device 300 rests directly on the skin surface of the user 108, the medicament delivery device 300 is prone to heating to near 98.6° F. For medicaments, like insulin, that are susceptible to elevated temperatures, the arrangement of the medicament delivery device 102 on the skin surface may affect the potency of the medicament. To that end, exemplary embodiments may determine the degree that the potency of the medicament has changed and compensate for the change in determining dosages of the medicament. The exemplary embodiments may provide multiple approaches for determining the degree that the potency of the medicament has changed and for compensating for the change, as will be detailed below.

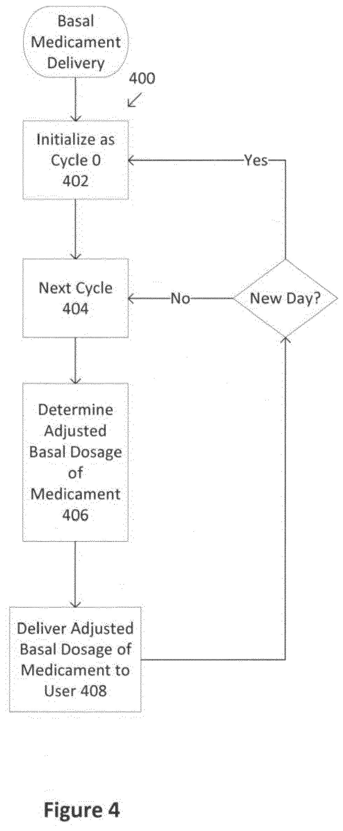

In many instances, a medicament delivery device 102 may be used to deliver medicament on an ongoing basis. The medicament delivery device 102 may deliver basal dosages at periodic intervals while in use. Figure 4 depicts an illustrative flowchart 400 of steps that may be performed to compensate for a change in potency of a medicament. The approach of the flowchart 400 assumes that medicament it is delivered at periodic intervals designated as cycles (e.g., 5-minute intervals) and compensates for changes in potency each cycle. The initial step 402 is to initialize a cycle counter at 0. The cycle counter maintains a count value of the current cycle and is incremented by one with each new cycle at 404. The control application 116 of the medicament delivery device 102 or the one or more control applications 120 of the management device 104 determine the adjusted basal dosage of medicament for the cycle at 406. The adjusted basal dosage is adjusted to compensate for changes in the potency of the medicament. The adjusted basal dosage of medicament is delivered to the user at 408. A check is then made at 410 of whether it is a new day or not. If not, the process is repeated for the next cycle at 404. If the check indicates a new day, the process repeats at 402 with setting the cycle counter to zero. The process may be repeated with each new day or, at 404, at each new cycle.

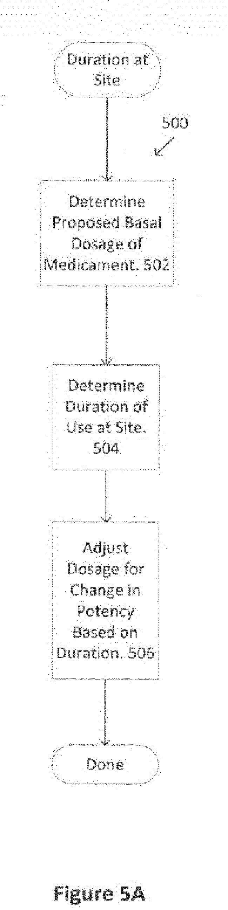

Figure 5A depicts a flowchart 500 of illustrative steps that may be performed in exemplary embodiments to adjust the dosage of the medicament to compensate for a change in potency based upon the duration that the medicament has been in the reservoir of the medicament delivery device 102 on the skin surface of the user 108. Initially, the control application 116 or 120 determines a proposed basal dosage of medicament at 502 using a control algorithm that does not account for change in potency. The proposed basal dosage of medicament may be determined by known control algorithms based on factors such as weight, age, past medicament delivery history, current glucose level, etc. This proposed basal dosage acts as a base dosage value that is adjusted to compensate for changes in potency of the medicament. In this instance, duration alone is the factor that determines the change in potency. As such, a determination is made of the duration of use at the site at 504. The control application 116 or 120 then adjust the dosage for the change in potency based on the duration at 506.

When the medicament is insulin, one approach to adjusting the basal dosage to compensate for changes in potency is captured by the following equation which adjusts the insulin dosage based on duration:

$$I_f(i) = I_{AID}(i) \cdot \left(1 + 0.006 \cdot \frac{i}{288}\right) \quad \text{(Equation 1)}$$

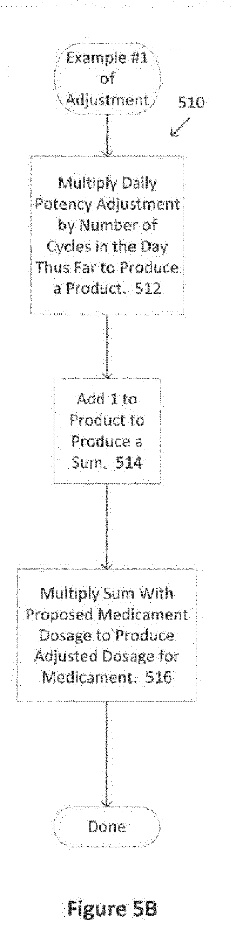

where $i$ is the current cycle, $I_f(i)$ is the compensated basal dosage of insulin at cycle $i$, and $I_{AID}(i)$ is the basal dosage the automated insulin delivery (AID) control algorithm would have delivered without compensation. Equation 1 is based on the notion that basal insulin is delivered at cycles of 5 minutes each so that there are 288 cycles per day. It has been shown that the potency of insulin may decrease by 18% over 28 days. That equates to a decrease of 0.6% a day. Other percentages of decrease values may be used. Thus, the potency decreases by 0.006/288 per cycle. Hence, $0.006 \times i/288$ in the above equation relates to how much the potency has decreased during the present day up to the current cycle. As shown in the flowchart 510 of Figure 5B, the daily potency adjustment (i.e., 0.006) is multiplied by the number of cycle in the day thus far (i.e., $i/288$) at 512. The value 1 is added to $0.006 \times i/288$ to compensate for the decrease in potency with an enlarged dosage relative to $I_{AID}(i)$ at 514. The resulting product is multiplied by $I_{AID}(i)$ to produce the adjusted dosage for the medicament at 516.

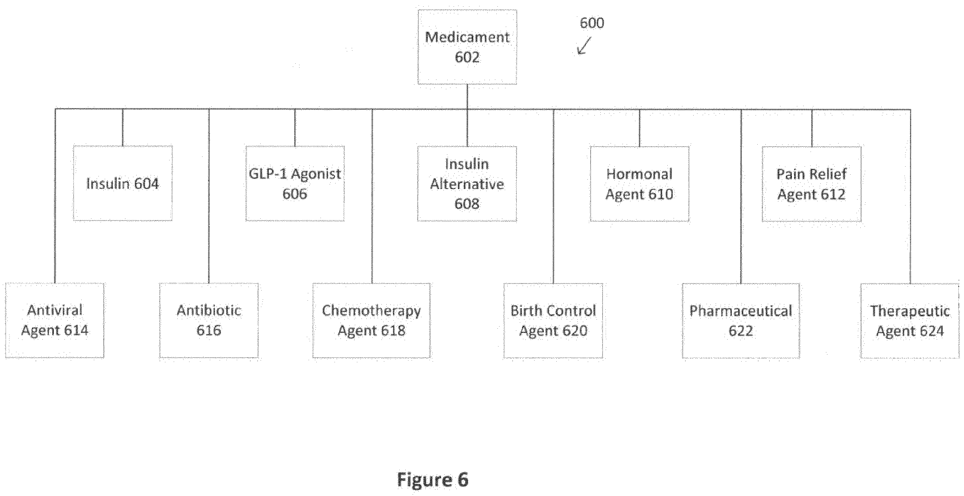

It should be appreciated that the above formulation of Equation 1 is particular to insulin. Other formulations may be necessitated for other medicaments. Figure 6 depicts a block diagram 600 showing various types of medicaments 602 to which the inventive aspects of this application may apply. As mentioned above, the medicament may be insulin 604, GLP-1 agonists 606, or other insulin alternatives 608. In addition, the medicament 602 may be a hormonal agent 610 or a pain relief agent 612. The medicament 602 may be an antiviral agent 614 or an antibiotic 616. The medicament 602 may be a chemotherapy agent 618 or a birth control agent 620 or a fertility agent. More generally, the medicament 602 may be any type of pharmaceutical 622 or therapeutic agent 624.

It should be appreciated that the potency of some medicaments may increase rather decrease over time or in response to other factors. In such instances, the basal delivery dosage should be decreased to compensate for the increase in potency.

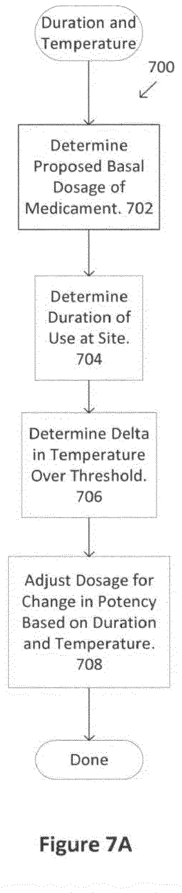

In an alternate approach, the temperature and duration may both be considered in determining changes in the potency of a medicament. Figure 7A depicts a flowchart 700 of illustrative steps that may be performed when adjusting for changes in potency due to both duration and temperature. Initially, the proposed basal dosage of medicament is determined per the control algorithm of the medicament delivery device 102 at 702. The duration that the medicament has been at the site on the user 108 is determined at 704. The delta in temperature relative to a threshold is determined at 706. For example, it is recommended that insulin be stored in the temperature range between 77°F and 86°F. One choice of a temperature threshold for insulin is 77°F, the lower bound of the acceptable temperature range. The dosage recommended by the control algorithm may be adjusted to compensate for a change in potency based on the determined duration and temperature values at 708.

A suitable equation for this approach where the medicament is insulin is:

$$I_f(i) = I_{AID}(i) \cdot \left(1 + 0.006 \cdot \frac{\max(0, (T(i) - 77))}{21.6} \cdot \frac{i}{244}\right) \quad \text{(Equation 2)}$$

where $i$ is the current cycle, $I_f(i)$ is the compensated basal dosage of insulin at cycle $i$, $I_{AID}(i)$ is the basal dosage the automated insulin delivery (AID) control algorithm of control application 116 or 120 would have delivered without compensation, max() is a maximum function that returns a maximum value and $T(i)$ is the current temperature in Fahrenheit at cycle $i$. The value 21.6 is the difference between the body temperature of the user 98.6°F and the lower bound of the acceptable temperature range 77°F. This approach assumes that there is some decay for any temperature above the lower threshold but not at or below the threshold. In addition, this approach assumes that the magnitude of the elevated temperature relative to the threshold affects the magnitude of loss potency. In other words, higher temperatures produce greater loss in potency. Thus, the loss in potency is based on both temperature and duration.

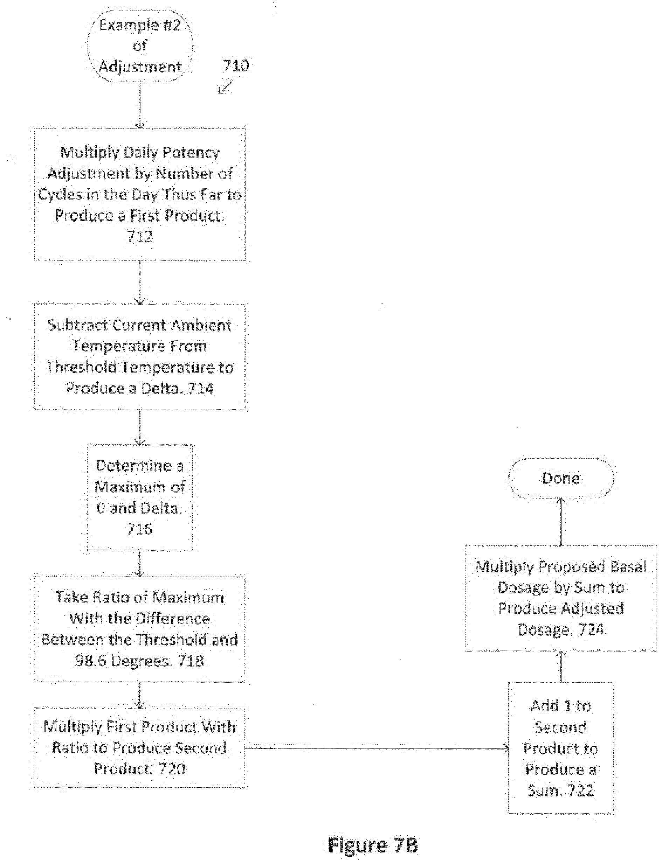

Figure 7B depicts a flowchart 710 of illustrative steps that may be performed when using Equation 2 to compensate for temperature and duration. At 712, the daily potency adjustment (i.e., 0.006) is multiplied by the number of cycles in the day thus far ($i/288$) to produce the first product. At 714, the current ambient temperature (i.e., $T(i)$) is subtracted from a threshold temperature (i.e., 77°F) to produce a delta. At 716, a maximum of zero and the calculated delta is determined (i.e., $\max(0, T(i)-77)$). At 718, the ratio of the maximum relative to the difference between the threshold and 98.6 is calculated (i.e., $\max(0, T(i)-77)/21.6$). At 720, the first product and the ratio are then multiplied to produce a second product. At 722, the second product is added to 1 to produce a sum. At 724, the proposed the basal dosage (i.e., $I_{AID}(i)$) is multiplied by the sum to produce the adjusted dosage.

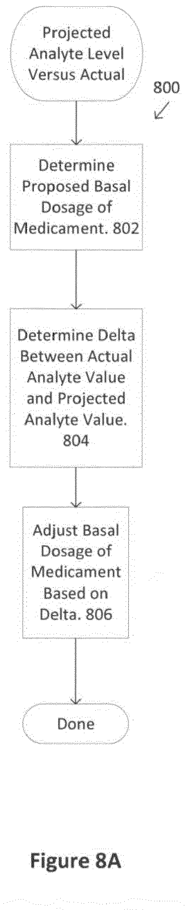

A third approach to compensating for changes in potency of a medicament involves looking at a projected analyte level versus an actual analyte level. Figure 8A depicts a flowchart 800 of illustrative steps that may be performed in exemplary embodiments to adopt this approach. Initially, at 802, a proposed basal dosage of medicament is determined. At 804, a delta value for the difference between the actual analyte value and the projected analyte value is determined. The difference between the projected analyte value in the actual analyte value is reflective of the change in potency of the medicament. For example, if the medicament is insulin, the analyte is glucose concentration and the most recent glucose concentration value is higher than the expected target glucose concentration value, this may be an indication that the potency of the insulin in the medicament delivery device has decreased. At 806, the basal dose of medicament is adjusted based upon the determined delta. The control application 116 or the control application 120 may figure out how much additional insulin needs to be delivered in the basal dosage to compensate for the residuals between actual glucose concentration value and target glucose concentration value.

It should be appreciated that these differences in actual glucose concentration value versus projected glucose concentration value may vary significantly based on the user's meal ingestion's and based upon location of the medicament delivery device. That said, the average deviation should not change as such, long-term changes in residuals between predictions and actual values may be attributed to the reduction and insulin potency or other medicament delivery site issues. Thus, the exemplary embodiments may rely upon the longer-term change in residuals in determining the potency variable *p*.

Where the medicament is insulin and the analyte values are glucose concentration values, the adjusted basal medicament dosage may be calculated as:

$$I_f(i) = I_{AID}(i) \cdot \left(1 + p \cdot \frac{i}{288}\right) \qquad \text{(Equation 3)}$$

where *p* is a parameter rather than 0.006 that represents the reduction in insulin potency each cycle. By using the parameter *p,* this approach allows more customization per medicament delivery device and per user.

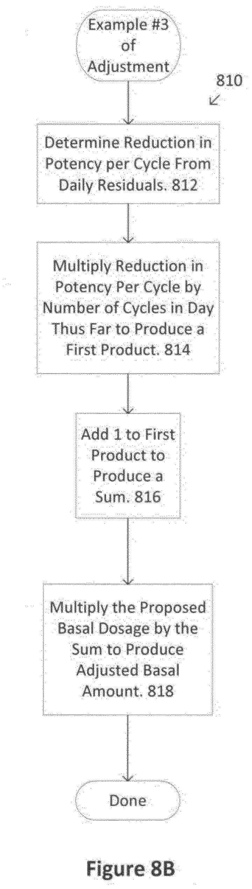

Figure 8B depicts a flowchart 810 of illustrative steps that may be performed in exemplary embodiment to compensate for changes in potency reflected in the difference between actual analyte levels and projected analyte levels. At 812, the reduction in potency per cycle (i.e., *p*) is determined. At 814, the reduction in potency *p* is multiplied by the number of cycles in the day thus far to produce a first product. At 816, the first product is added to 1. At 818, the resulting sum is multiplied by $I_{AID}(i)$.

As part of determining a suitable value for *p,* the exemplary embodiments may calculate the average daily residuals between the projected glucose concentration values in the actual glucose concentration values. A suitable equation for calculating the average daily residuals is:

$$\bar{R} = \frac{\sum_{i=1}^{288} d_i}{288} \qquad \text{(Equation 4)}$$

where $\bar{R}$ represents a daily average of the residuals. The value $d_i$ may be calculated as:

$$d_i = \sum_{k=1}^{12} G(i-k) - p_{i-12}(k) \qquad \text{(Equation 5)}$$

Where $G(i-k)$ is the actual glucose concentration value at cycle *i - k* and $p_{i-12}(k)$ is the last available prediction of the glucose concentration value (i.e., the projected glucose concentration value) twelve cycles prior to the current cycle *i*. The prediction horizon is 12 cycles. Where the cycles are 5 minutes in length, the prediction horizon is one hour.

Next, the reduction in potency in the insulin for *j*th day may be determined by calculating the change in daily residuals as:

$$\bar{R}_j = \frac{\sum_{i=244(j-1)}^{288j} d_i}{288} \qquad \text{(Equation 6)}$$

The control application 116 or 120 may then adjust the basal insulin dosages based on the determined reduction in potency. The potency for day 1 may be calculated as:

$$p_j = min\left(1.006, max\left(1.1 + \frac{\bar{R}_j - \bar{R}_1}{\bar{R}_1}\right)\right)$$

This value $p_j$ may serve as a baseline for calculating the potency $p$.

While exemplary embodiments have been described herein, various changes in form and detail relative to the exemplary embodiments may made without departing from the intended scope of the claims as appended hereto.

Although the present invention is defined in the attached claims, it should be understood that the present invention can also (alternatively) be defined in accordance with the following embodiments:

1. A method performed by a processor of an electronic device, comprising:

calculating a basal dosage of a medicament to be delivered to a user by a medicament delivery device;

determining an amount that at least one factor affects a potency of the medicament; and

adjusting the calculated basal dosage of the medicament to compensate for the determined amount that the at least one factor has affected the potency of the medicament.

2. The method of embodiment 1, wherein the medicament is insulin.

3. The method of one of the preceding embodiments, wherein the at least one factor comprises temperature, exposure to light or exposure to air.

4. The method of one of the preceding embodiments, wherein the at least one factor comprises a duration of time that the medicament has been stored in the medicament delivery device.

5. The method of one of the preceding embodiments, wherein the at least one factor decreases the potency of the medicament and the calculated dosage is adjusted to a larger dosage to compensate.

6. The method of one of the preceding embodiments, wherein the at least one factor comprises temperature, an average temperature or a histogram of temperatures and a duration of time that the medicament has been stored in the medicament delivery device at a particular temperature or at a particular average temperature.

7. The method of one of the preceding embodiments, wherein the adjusting comprises adjusting the calculated basal dosage based on how much a current temperature is above a target temperature.

8. The method of one of the preceding embodiments, wherein the adjusting comprises adjusting the calculated basal dosage based on how much an actual blood glucose level compares with a projected blood glucose level.

9. A medicament delivery device, comprising:

a medicament storage;

a pump for pumping medicament out of the storage for delivery to a user;

a storage medium for storing data and computer programming instructions;

a processor for executing the computer programming instructions, the computer programming instructions causing the processor to perform the following:

calculating a basal dosage of a medicament to be delivered to a user by the medicament delivery device;

determining an amount that at least one factor affects a potency of the medicament; and

adjusting the calculated basal dosage of medicament to compensate for the determined amount that the at least one factor has affected the potency of the medicament.

10. The medicament delivery device of embodiment 9, wherein the computer programming instructions additionally cause the processor to control the pump to deliver the adjusted basal dosage of medicament to the user.

11. The medicament delivery device of one of embodiments 9 or 10, wherein the medicament is insulin.

12. The medicament delivery device of one of embodiments 9 to 11, wherein the at least one factor comprises temperature.

13. The medicament delivery of one of embodiments 9 to 12, wherein the at least one factor comprises a duration of time that the medicament has been stored in the medicament delivery device.

14. The medicament delivery device of one of embodiments 9 to 13, wherein the at least one factor comprises temperature and a duration of time that the medicament has been stored in the medicament delivery device.

15. The medicament delivery device of one of embodiments 9 to 14, wherein the adjusting comprises adjusting the calculated basal dosage based on how much an average temperature is above a target temperature.

16. The medicament delivery device of one of embodiments 9 to 15, wherein the adjusting comprises adjusting the calculated basal dosage based on how much an actual blood glucose level compares with a projected blood glucose level.

17. A method performed by a processor in an on-body insulin delivery device, comprising:

calculating a basal dosage for a user based on at least a current blood glucose level;

calculating how much a potency of the insulin has decreased over a time period; and

increasing the calculated basal dosage of the insulin to offset the calculated decrease in potency of the insulin.

18. The method of embodiment 17, wherein calculating how much the potency of the insulin has decreased over the time period comprises determining a temperature for the insulin and adjusting for the determined temperature if the determined temperature exceeds a threshold.

19. The method of one of embodiments 17 or 18, further comprising determining a delta of how much the determined temperature exceeds the threshold and a magnitude of the adjustment for the determined temperature is based on a magnitude of the delta.

20. The method of one of embodiments 17 to 19, wherein calculating how much the potency of the insulin has decreased over the time period comprises determining a delta between a projected blood glucose level for the user and an actual blood glucose level for the user and determining how much the potency of the insulin has decreased based on a magnitude of the determined delta.

**Claims**

1. A method performed by a processor of an electronic device, comprising:

calculating a basal dosage of a medicament to be delivered to a user by a medicament delivery device;
determining an amount that at least one factor affects a potency of the medicament; and
adjusting the calculated basal dosage of the medicament to compensate for the determined amount that the at least one factor has affected the potency of the medicament.

2. The method of claim 1, wherein the medicament is insulin.

3. The method of one of the preceding claims, wherein the at least one factor comprises temperature, exposure to light or exposure to air.

4. The method of one of the preceding claims, wherein the at least one factor comprises a duration of time that the medicament has been stored in the medicament delivery device.

5. The method of one of the preceding claims, wherein the at least one factor decreases the potency of the medicament and the calculated dosage is adjusted to a larger dosage to compensate.

6. The method of one of the preceding claims, wherein the at least one factor comprises temperature, an average temperature or a histogram of temperatures and a duration of time that the medicament has been stored in the medicament delivery device at a particular temperature or at a particular average temperature.

7. The method of one of the preceding claims, wherein the adjusting comprises adjusting the calculated basal dosage based on how much a current temperature is above a target temperature.

8. The method of one of the preceding claims, wherein the adjusting comprises adjusting the calculated basal dosage based on how much an actual blood glucose level compares with a projected blood glucose level.

9. A medicament delivery device, comprising:

   a medicament storage;
   a pump for pumping medicament out of the storage for delivery to a user;
   a storage medium for storing data and computer programming instructions;
   a processor for executing the computer programming instructions, the computer programming instructions causing the processor to perform the following:

   calculating a basal dosage of a medicament to be delivered to a user by the medicament delivery device;
   determining an amount that at least one factor affects a potency of the medicament; and
   adjusting the calculated basal dosage of medicament to compensate for the determined amount that the at least one factor has affected the potency of the medicament.

10. The medicament delivery device of claim 9, wherein the computer programming instructions additionally cause the processor to control the pump to deliver the adjusted basal dosage of medicament to the user.

11. The medicament delivery device of one of claims 9 or 10, wherein the medicament is insulin.

12. The medicament delivery device of one of claims 9 to 11, wherein the at least one factor comprises temperature.

13. The medicament delivery of one of claims 9 to 12, wherein the at least one factor comprises a duration of time that the medicament has been stored in the medicament delivery device.

14. The medicament delivery device of one of claims 9 to 13, wherein the at least one factor comprises temperature and a duration of time that the medicament has been stored in the medicament delivery device.

15. A method performed by a processor in an on-body insulin delivery device, comprising:

   calculating a basal dosage for a user based on at least a current blood glucose level;
   calculating how much a potency of the insulin has decreased over a time period; and
   increasing the calculated basal dosage of the insulin to offset the calculated decrease in potency of the insulin.

Figure 1

**Figure 2**

302

Pad

304

Reservoir

Medicament
Delivery Device

300

306

**Figure 3**

Figure 4

```
                    ┌─────────────┐
                    │  Duration at │
                    │     Site     │
                    └─────────────┘
                           │              500
                           │              ↙
                           ▼
                  ┌──────────────────┐
                  │    Determine     │
                  │  Proposed Basal  │
                  │    Dosage of     │
                  │  Medicament. 502 │
                  └──────────────────┘
                           │
                           ▼
                  ┌──────────────────┐
                  │    Determine     │
                  │   Duration of    │
                  │   Use at Site.   │
                  │       504        │
                  └──────────────────┘
                           │
                           ▼
                  ┌──────────────────┐
                  │     Adjust       │
                  │   Dosage for     │
                  │    Change in     │
                  │     Potency      │
                  │    Based on      │
                  │  Duration. 506   │
                  └──────────────────┘
                           │
                           ▼
                    ┌─────────────┐
                    │    Done     │
                    └─────────────┘
```

**Figure 5A**

Example #1 of Adjustment

510

Multiply Daily Potency Adjustment by Number of Cycles in the Day Thus Far to Produce a Product. 512

Add 1 to Product to Produce a Sum. 514

Multiply Sum With Proposed Medicament Dosage to Produce Adjusted Dosage for Medicament. 516

Done

Figure 5B

Figure 6

Figure 7A

Example #2
of
Adjustment
710

Multiply Daily Potency
Adjustment by Number of
Cycles in the Day Thus Far to
Produce a First Product.
712

Subtract Current Ambient
Temperature From
Threshold Temperature to
Produce a Delta. 714

Determine a
Maximum of
0 and Delta.
716

Take Ratio of Maximum
With the Difference
Between the Threshold and
98.6 Degrees. 718

Multiply First Product With
Ratio to Produce Second
Product. 720

Add 1 to
Second
Product to
Produce a
Sum. 722

Multiply Proposed Basal
Dosage by Sum to
Produce Adjusted
Dosage. 724

Done

**Figure 7B**

Projected
Analyte Level
Versus Actual

800

Determine
Proposed Basal
Dosage of
Medicament. 802

Determine Delta
Between Actual
Analyte Value
and Projected
Analyte Value.
804

Adjust Basal
Dosage of
Medicament
Based on
Delta. 806

Done

Figure 8A

Example #3
of
Adjustment

810

Determine Reduction in
Potency per Cycle From
Daily Residuals. 812

Multiply Reduction in
Potency Per Cycle by
Number of Cycles in Day
Thus Far to Produce a
First Product. 814

Add 1 to First
Product to
Produce a
Sum. 816

Multiply the Proposed
Basal Dosage by the
Sum to Produce
Adjusted Basal
Amount. 818

Done

Figure 8B

European Patent Office

Office européen des brevets

Europäisches Patentamt

**EUROPEAN SEARCH REPORT**

Application Number

EP 22 19 2281

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2003/018304 A1 (SAGE BURTON H [US]) 23 January 2003 (2003-01-23) * abstract * * paragraph [0010] - paragraph [0022] * * paragraph [0025] - paragraph [0068] * * claims 1,2,4,12-14,18-21 * * figure 1 * ----- | 1-15 | INV. G16H20/17 G16H40/63 A61M5/14 |
| X | WO 2021/059211 A1 (JANSSEN PHARMACEUTICALS INC [US]) 1 April 2021 (2021-04-01) * abstract * * paragraph [0007] - paragraph [0025] * * paragraph [0074] - paragraph [0089] * * paragraph [0105] - paragraph [0113] * * paragraph [0133] - paragraph [0226] * * claims 1-6,11,24-26 * * figures 1,2,8 * ----- | 1-15 | |
| | | | TECHNICAL FIELDS SEARCHED (IPC) G16H A61M |

The present search report has been drawn up for all claims

| Place of search | Date of completion of the search | Examiner |
|---|---|---|
| Munich | 13 January 2023 | Hilbig, Matthias |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 22 19 2281

This annex lists the patent family members relating to the patent documents cited in the above-mentioned European search report.
The members are as contained in the European Patent Office EDP file on
The European Patent Office is in no way liable for these particulars which are merely given for the purpose of information.

13-01-2023

| Patent document cited in search report | Publication date | Patent family member(s) | Publication date |
|---|---|---|---|
| US 2003018304 A1 | 23-01-2003 | NONE | |
| WO 2021059211 A1 | 01-04-2021 | AU 2020351853 A1 | 19-05-2022 |
| | | BR 112022005657 A2 | 19-07-2022 |
| | | CA 3155914 A1 | 01-04-2021 |
| | | CN 114761059 A | 15-07-2022 |
| | | EP 4034204 A1 | 03-08-2022 |
| | | IL 291608 A | 01-05-2022 |
| | | JP 2022549668 A | 28-11-2022 |
| | | KR 20220088703 A | 28-06-2022 |
| | | WO 2021059211 A1 | 01-04-2021 |

EPO FORM P0459

For more details about this annex : see Official Journal of the European Patent Office, No. 12/82